Europäisches Patentamt

European Patent Office

Office européen des brevets

(11) Veröffentlichungsnummer : **0 190 536**
**B1**

(12) **EUROPÄISCHE PATENTSCHRIFT**

(45) Veröffentlichungstag der Patentschrift :
25.05.88

(51) Int. Cl.⁴ : **C 09 K 11/80, C 09 K 11/78**

(21) Anmeldenummer : **85810047.2**

(22) Anmeldetag : **07.02.85**

(54) **Leuchtstofflampe.**

(43) Veröffentlichungstag der Anmeldung :
**13.08.86 Patentblatt 86/33**

(45) Bekanntmachung des Hinweises auf die Patenterteilung : **25.05.88 Patentblatt 88/21**

(84) Benannte Vertragsstaaten :
**AT BE CH DE FR GB IT LI LU NL SE**

(56) Entgegenhaltungen :
**FR-A- 2 539 555**

(73) Patentinhaber : **Wolff, Friedrich**
**Störklingasse 38**
**CH-4125 Riehen/Basel (CH)**

(72) Erfinder : **Wolff, Friedrich**
**Störklingasse 38**
**CH-4125 Riehen/Basel (CH)**

(74) Vertreter : **Eschmann, Heinz et al**
**A. Braun, Braun, Héritier, Eschmann AG Patentanwälte Holbeinstrasse 36-38**
**CH-4051 Basel (CH)**

## Beschreibung

Die Erfindung betrifft eine UV-Leuchtstofflampe mit spezifischen photobiologischen Wirkungen und dementsprechend einem spektralen Energieverteilungsverhältnis zwischen UVA und UVB Von 100-250 zu 1.

Es sind Leuchtstofflampen bekannt, deren Leuchtstoff im UVA-Bereich Strahlungen emittiert und deren Filter die UVC-Strahlung sowie mindestens den kurzwelligen Teil der UVB-Strahlung abfiltert. Gemäss einer Ausführung dieser bekannten Lampen besteht der Leuchtstoff aus Barium-Metasilicat, aktiviert mit Blei. Diese Lampen erzielen wohl die biologisch erwünschten Wirkungen, sind aber mit dem Nachteil verbunden, dass sie eine kurze Nutzlebensdauer aufweisen.

Eine andere, ebenfalls für Bestrahlungszwecke einsetzbare Leuchtstofflampe ist mit einer im wesentlichen UVA-Strahlung emittierenden Leuchtstoffschicht Versehen, welche aus Cerium-Strontium-Magnesium-Aluminat besteht. Diese Lampe besitzt zwar den Vorteil einer längeren Nutzlebensdauer bei Erzielung der erwünschten biologischen Wirkungen, doch ist die Strahlungsausbeute im UV-Bereich gering, da der verwendete Leuchtstoff ein relativ breites Spektrum aufweist, welches bis in den sichtbaren Bereich reicht.

Durch die DE-OS 3 400 385 ist ferner eine Niederdruckquecksilberdampfentladungslampe bekannt, deren Leuchtstoffschicht einen Leuchtstoff auf der Basis eines mit zweiwertigem Europium aktivierten Strontium-Tetraborats enthält. Dadurch ergibt sich einerseits zwar im Vergleich zu den vorgenannten Leuchtstofflampen eine besonders lange Nutzlebensdauer bei konstant bleibend hoher Strahlungsausbeute ; andererseits erzeugt diese Lampe jedoch praktisch nur Strahlung im UVA-Bereich und kann somit die biologisch erwünschten Wirkungen nur teilweise erzielen.

Das von UV-Lampen abgestrahlte Licht ist von Natur aus blau, was auf das vegetative Nervensystem dämpfend wirkt. Eine Bestrahlung mit solchen Lampen kann deshalb zu unerwünschter Schlappheit führen.

Es ist die Aufgabe der vorliegenden Erfindung, eine Leuchtstofflampe der einleitend erwähnten Art vorzuschlagen, welche
die biologisch erwünschten Wirkungen hervorbringt und demgemäss ein spektrales Energieverteilungsverhältnis zwischen UVA und UVB von 100-250 zu 1 aufweist,
eine lange Nutzlebensdauer hat und
sich durch eine hohe Strahlungsausbeute auszeichnet.

Dabei sollte möglichst die von Natur aus blaue Lichtfarbe durch Zumischung eines geeigneten, rotes Licht erzeugenden Leuchtstoffes modifiziert werden, welcher kein UVA absorbiert, sondern nach Möglichkeit UVA-Strahlung emittieren sollte.

Diese Aufgabe wird durch die im Patentanspruch 1 definierte Erfindung gelöst. Bevorzugte Ausführungsformen ergeben sich aus den abhängigen Ansprüchen.

Die Lösung der Aufgabe ergibt sich, wenn man das Cerium-Strontium-Magnesium-Aluminat und das mit zweiwertigem Europium aktivierte Strontium-Tetraborat in geeignetem Verhältnis kombiniert. Eine bevorzugte Mischung enthält zwischen 45 und 55 % Cerium-Strontium-Magnesium-Aluminat und zwischen 55 und 45 % Strontium-Tetraborat, das mit zweiwertigem Europium aktiviert wurde. Eine besonders gute Kombination hat sich ergeben, wenn diese beiden Substanzen im Verhältnis 1 : 1 gemischt werden.

Gemäss einer weiteren, bevorzugten Variante enthält der Leuchtstoff je 48 % der beiden vorerwähnten Substanzen mit 4 % Yttrium das mit Europium aktiviert wurde. Die dadurch erzielte rötlich-weisse Lichtfarbe verhindert eine dämpfende Wirkung auf das vegetative Nervensystem und damit auch die bereits erwähnte Schlappheit nach der Bestrahlung.

Das beiliegende Diagramm zeigt den Vergleich der spektralen Energieverteilung in Abhängigkeit von der Wellenlänge. Verglichen wurden die Spektralkurven a und b zweier bekannter Leuchtstoffe mit der Spektralkurve c eines erfindungsgemässen Leuchtstoffes. Demnach bezieht sich
die Kurve a auf einen Leuchtstoff aus Strontium-Tetraborat, das mit zweiwertigem Europium aktiviert wurde,
die Kurve b auf einen Leuchtstoff aus Cerium-Strontium-Magnesium-Aluminat und
die Kurve c auf einen erfindungsgemässen Leuchtstoff, welcher 50 % Cerium-Strontium-Magnesium-Aluminat und 50 % Strontium-Tetraborat, das mit zweiwertigem Europium aktiviert wurde, enthielt.

Die Kurve c zeigt eine noch immer grosse Strahlungsausbeute im UVA-Bereich, bei gleichzeitig geringem, im gewünschten Verhältnis feststellbaren UVB-Anteil. Die Nutzlebensdauer dürfte bei den verglichenen Lampen etwa gleich lang sein.

Dank der gewählten Leuchtstoffkombination ergibt sich somit überraschenderweise bei langer Nutzlebensdauer eine Lampe, welche die erwünschten photobiologischen Wirkungen in einem optimalen Ausmasse zu erzielen gestattet.

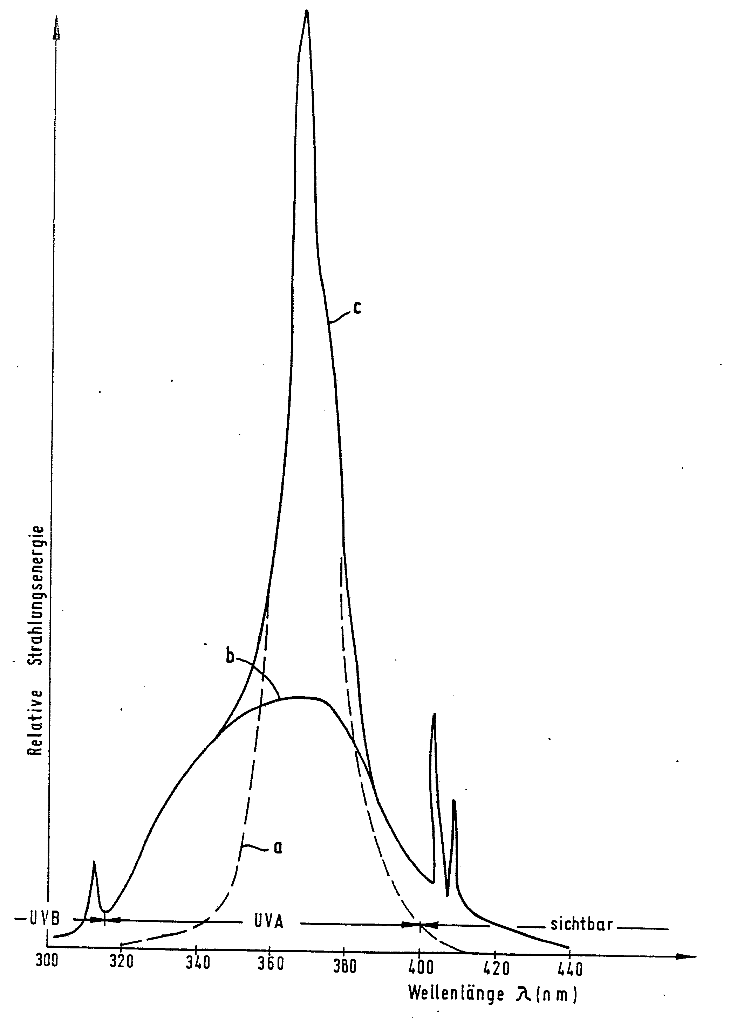

## Patentansprüche

1. Leuchtstofflampe mit spezifischen photobiologischen Wirkungen und dementsprechend einem spektralen Energieverteilungsverhältnis zwischen UVA und UVB von 100-250 zu 1, gekennzeichnet durch einen Leuchtstoff, welcher Cerium-Strontium-Magnesium-Aluminat und Strontium-Tetraborat, das. mit zweiwertigem Europium aktiviert ist, enthält.

2. Leuchtstofflampe nach Anspruch 1, dadurch gekennzeichnet, dass der Leuchtstoff zwischen 45 und 55 % Cerium-Strontium-Magnesium-Aluminat und zwischen 55 und 45 % Strontium-Tetraborat, das mit zweiwertigem Europium aktiviert ist, enthält.

3. Leuchtstofflampe nach Anspruch 1, dadurch gekennzeichnet, dass der Leuchtstoff 48 % Cerium-Strontium-Magnesium-Aluminat, 48 % Strontium-Tetraborat, das mit zweiwertigem Europium aktiviert ist und 4 % Yttrium, das mit Europium aktiviert wurde, enthält.

**Claims**

1. Fluorescent lamp having specific photobiological effects and accordingly a spectral energy distribution ratio of UVA to UVB of 100-250 : 1, characterized by a fluorescent material which contains cerium strontium magnesium aluminate and strontium tetraborate which is activated with divalent europium.

2. Fluorescent lamp according to Claim 1, characterized in that the fluorescent material contains between 45 and 55 % of cerium strontium magnesium aluminate and between 55 and 45 % of strontium tetraborate which is activated with divalent europium.

3. Fluorescent lamp according to Claim 1, characterized in that the fluorescent material contains 48 % of strontium tetraborate which is activated with divalent europium, and 4 % of yttrium which has been activated with europium.

**Revendications**

1. Lampe fluorescente ayant des effets photo-biologiques spécifiques et, par conséquent, un rapport de distribution de l'énergie spectrale entre UVA et UVB de 100-250 à 1, caractérisée par une substance fluorescente qui contient de l'aluminate de cérium-strontium-magnésium et du tétraborate de strontium qui a été activé par de l'europium divalent.

2. Lampe fluorescente suivant la revendication 1, caractérisée en ce que la substance fluorescente contient entre 45 et 55 % d'aluminate de cérium-strontium-magnésium et entre 55 et 45 % de tétraborate de strontium qui a été activé par de l'europium divalent.

3. Lampe fluorescente suivant la revendication 1, caractérisée en ce que la substance fluorescente contient 45 % d'aluminate de cérium-strontium-magnésium, 45 % de tétraborate de strontium qui ⌐té activé par de l'europium divalent et 4% d'y ⌐ium qui a été activé par de l'europium.

**0 190 536**